# EUROPÄISCHE PATENTSCHRIFT

(11) **EP 1 286 957 B1**
(45) Veröffentlichungstag und Bekanntmachung des Hinweises auf die Patenterteilung: **03.11.2004**
(21) Anmeldenummer: 01938186.2
(22) Anmeldetag: 07.05.2001
(51) Int. Cl.: C07C 271/28, A61K 31/325, A61P 5/16

(54) **DIPHENYLMETHANDERIVATE**
DIPHENYLMETHANE DERIVATIVES
DERIVES DE DIPHENYLMETHANE

(30) Priorität: 19.05.2000 DE 10024939
(43) Veröffentlichungstag der Anmeldung: 05.03.2003
(73) Patentinhaber: Bayer HealthCare AG, 51368 Leverkusen (DE)
(72) Erfinder: HANING, Helmut, Milford, CT 06460 (US); SCHMIDT, Gunter, 42115 Wuppertal (DE); PERNERSTORFER, Josef, 42103 Wuppertal (DE); BISCHOFF, Hilmar, 42113 Wuppertal (DE); SCHMECK, Carsten, 42119 Wuppertal (DE); VÖHRINGER, Verena, 42113 Wuppertal (DE); WOLTERING, Michael, 42105 Wuppertal (DE); KRETSCHMER, Axel, 42113 Wuppertal (DE); FAESTE, Christiane, 42781 Haan (DE)
(86) Internationale Anmeldenummer: PCT/EP2001/005142
(87) Internationale Veröffentlichungsnummer: WO 2001/090053

(56) Entgegenhaltungen:
- EP-A- 0 188 351
- EP-A- 0 580 550
- WO-A-98/57919
- WO-A-99/26966

## Beschreibung

Die Erfindung betrifft neue Diphenylmethanderivate, Verfahren zur ihrer Herstellung sowie ihre Verwendung in Arzneimitteln, insbesondere für die Indikationen Arteriosklerose und Hypercholesterolämie.

In der europäischen Anmeldung 580 550 A werden Oxamsäurederivate beschrieben, die cholesterolsenkende Eigenschaften in Säugetieren besitzen. In dieser Anmeldung wird ein in-vitro-Test beschrieben, der auf der Bindung an Thyroid-Hormon-Zellrezeptoren (sogenannte T₃-Nuklear-Rezeptoren) aufbaut. Für einige der dort beschriebenen Verbindungen werden IC-50-Werte von 0,2 nM und 0,1 nM bei dem L-Trüodothyronine (LT₃)-Nuklear-Rezeptor-Test angegeben. Als pharmakologische Eigenschaft wird die Reduktion von Plasma-Cholesterol, insbesondere von LDL-Cholesterol hervorgehoben. Cholesterolsenkende Wirkungen werden auch in der europäischen Anmeldung EP-A-188 351 beschrieben für bestimmte Diphenylether mit Thyroid-Hormon-ähnlichen Wirkungen.

Tripp et al. in J. Med. Chem. 1973, 16(1), 60-64 beschreiben die Synthese von Methylen- und Carbonyl-verbrückten Analoga des Iodthyronins. Sie stellten fest, dass deren thyromimetische Aktivität geringer war, als die der entsprechenden O-oder S-verbrückten Verbindungen.

Auch Psychoyos et al. in Endocrinology 1973, 92(1), 243-250 untersuchten die thyromimetische Aktivität von Methylen-verbrückten Thyroidhormon-Analoga. Sie kamen ebenfalls zu dem Ergebnis, dass die Methylen-verbrückten Verbindungen im Vergleich zu den O-verbrückten Verbindungen weniger potent waren.

WO 98/57919 offenbart selektive Thyroidhormon-Agonisten mit Diphenylmethan-Grundstruktur und ihre Verwendung in Arzneimitteln.

WO 99/26966 beschreibt neue Methoden für das Design von Liganden, die an bestimmte Rezeptoren (sog. *"nuclear receptors*") binden. Insbesondere wird beschrieben, dass Moleküle, die sich zur Modulation des Thyroidrezeptors eignen, bestimmten dreidimensionalen Anforderungen genügen müssen.

Die Erfindung betrifft Diphenylmethanderivate der allgemeinen Formel (I) 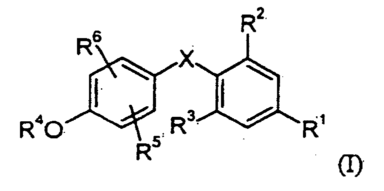 worin
- X: CH₂, CHF oder CF₂ bedeutet,
- R¹: für eine Gruppe der Formel -NH-C(O)-C(O)-OR¹¹ steht,
worin
- R¹¹: Wassertoff oder (C₁-C₄)-Alkyl bedeutet,
- R² und R³: gleich oder verschieden sind und für Halogen, (C₁-C₄)-Alkyl, Cyclo-propyl, CF₃, CHF₂ oder CH₂F stehen,
- R⁴: Wasserstoff oder (C₁-C₃)-Alkyl bedeutet,
- R⁵: Wasserstoff bedeutet,
und
- R⁶: für (C₁-C₆)-Alkyl oder (C₃-C₆)-Cycloalkyl steht oder für einen Rest der Formel 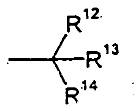 steht,
worin
R¹² für Phenyl, Pyrimidinyl, Pyridyl oder 3(2*H*)-Pyridazinonyl, die jeweils bis zu dreifach gleich oder verschieden durch Halogen, Hydroxy, Trifluormethyl, (C₁-C₃)-Alkyl oder (C₁-C₃)-Alkoxy substituiert sein können, oder für einen Rest der Formel -NR¹⁸R¹⁹ steht, worin
R¹⁸ und R¹⁹ gleich oder verschieden sind und unabhängig voneinander für (C₁-C₄)-Alkyl stehen, das durch Hydroxy, Amino, Mono- oder Di-(C₁-C₄)-alkylamino, oder durch Phenyl, Pyridyl oder Pyrimidinyl, die jeweils gegebenenfalls bis zu dreifach durch Hydroxy, Halogen, Trifluor-methyl, Methoxy oder (C₁-C₃)- Alkyl substituiert sind, substituiert sein kann,
R¹³ und R¹⁴ gemeinsam für eine Oxo-Gruppe stehen oder gleich oder verschieden sind und Wasserstoff, Hydroxy, (C₁-C₆)-Alkyl, bedeuten,
und deren Salze.

Bevorzugt sind Verbindungen der Formel (I), in denen R² und R³ für Methyl stehen. Bevorzugt sind Verbindungen der Formel (I), in denen R⁵ für Wasserstoff stehen. Bevorzugt sind Verbindungen der Formel (I), in denen R⁴ für Methyl oder Wasserstoff stehen.

Als Heterocyclen in der Definition von R¹² und R¹⁷ bis R²⁴ seien vorzugsweise genannt:

Ein 5- bis 8-gliedriger gesättigter, teilweise ungesättigter oder aromatischer gegebenenfalls benzokondensierter Heterocyclus mit bis zu 4 Heteroatomen aus der Reihe S, N und/oder O, d.h. ein Heterocyclus, der eine oder mehrere Doppelbindungen enthalten kann und der über ein Ringkohlenstoffatom oder ein Ringstickstoffatom verknüpft ist. Beispielsweise seien genannt: Tetrahydrofur-2-yl, Tetrahydrofur-3-yl, Pyrrolidin-1-yl, Pyrrolidin-2-yl, Pyrrolidin-3-yl, Pyrrolin-1-yl, Piperidin-1-yl, Piperidin-3-yl, 1,2-Dihydropyridin-1-yl, 1,4-Dihydropyridin-1-yl, Piperazin-1-yl, Morpholin-1-yl, Azepin-1-yl, 1,4-Diazepin-1-yl, Furan-2-yl, Furan-3-yl, Pyrrol-1-yl, Pyrrol-2-yl, Pyrrol-3-yl, Thienyl, Thiazolyl, Oxazolyl, Imidazolyl, Triazolyl, Tetrazolyl, Pyridyl, Pyrimidyl, Pyrazinyl, Pyrimidinonyl, Pyridazinonyl.

Bevorzugt sind aus dieser Liste: Pyridyl, Pyrimidyl, Pyridazinyl, Pyrimidinonyl, Pyridazinonyl.

(C₁-C₈)-Alkyl, (C₁-C₆)-Alkyl, (C₁-C₄)-Alkyl und (C₁-C₃)-Alkyl stehen im Rahmen der Erfindung für einen geradkettigen oder verzweigten Alkylrest mit 1 bis 8, 1 bis 6 1 bis 4 bzw. 1 bis 3 Kohlenstoffatomen. Bevorzugt ist ein geradkettiger oder verzweigter Alkylrest mit 1 bis 3 Kohlenstoffatomen. Beispielsweise seien genannt: Methyl, Ethyl, n-Propyl, Isopropyl, t-Butyl, n-Pentyl und n-Hexyl.

(C₆-C₁₀)-Aryl steht im Rahmen der Erfindung für einen aromatischen Rest mit 6 bis 10 Kohlenstoffatomen. Bevorzugte Arylreste Phenyl und Naphthyl.

(C₃-C₈)-Cycloalkyl, (C₃-C₇)-Cycloalkyl und (C₃-C₆)-Cycloalkyl stehen im Rahmen der Erfindung für eine Cycloalkylgruppe mit 3 bis 8, 3 bis 7 bzw. 3 bis 6 Kohlen stoffatomen. Bevorzugt seien genannt: Cyclopropyl, Cyclobutyl, Cyclopentyl, Cyclohexyl und Cycloheptyl.

(C₁-C₆)-Alkoxy steht im Rahmen der Erfindung für einen geradkettigen oder verzweigten Alkoxyrest mit 1 bis 6 Kohlenstoffatomen. Bevorzugt ist ein geradkettiger oder verzweigter Alkoxyrest mit 1 bis 3 Kohlenstoffatomen. Beispielsweise seien genannt: Methoxy, Ethoxy, n-Propoxy, Isopropoxy, t-Butoxy, n-Pentoxy und n-Hexoxy.

(C₁-C₆)-Acyl steht im Rahmen der Erfindung insbesondere für einen geradkettigen oder verzweigten Alkylrest mit 1 bis 6 Kohlenstoffatomen, der in der 1-Position ein doppelt gebundenes Sauerstoffatom trägt und über die 1-Position verknüpft ist. Beispielsweise seien genannt: Formyl, Acetyl, Propionyl, n-Butyryl, i-Butyryl, Pivaloyl, n-Hexanoyl.

Halogen schließt im Rahmen der Erfindung Fluor, Chlor, Brom und Iod ein. Bevorzugt sind Fluor, Chlor oder Brom.

Die erfindungsgemäßen Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen in tautomeren Formen vorliegen. Dies ist dem Fachmann bekannt, und derartige Verbindungen sind ebenfalls vom Umfang der Erfindung umfasst.

Die erfindungsgemäßen Verbindungen können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen, oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbonoder Sulfonsäuren wie beispielsweise Essigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Physiologisch unbedenkliche Salze können ebenso Salze der erfindungsgemäßen Verbindungen mit Basen sein, wie beispielsweise Metall- oder Ammoniumsalze. Bevorzugte Beispiele sind Alkalimetallsalze (z.B. Natrium- oder Kaliumsalze), Erdalkalisalze (z.B. Magnesium- oder Calciumsalze), sowie Ammoniumsalze, die abgeleitet sind von Ammoniak oder organischen Aminen, wie beispielsweise Ethylamin, Di- bzw. Triethylamin, Ethyldiisopropylamin, Ethanolamin, Di- bzw. Triethanolamin, Dicyclohexylamin, Dimethylaminoethanol, Dibenzylamin, N-Methylmorpholin, Dihydroabietylamin, 1-Ephenamin, Methylpiperidin, Arginin, Lysin, Ethylendiamin oder 2-Phenylethylamin.

Die erfindung Verbindungen können auch in Form ihrer Solvate insbesondere in Form ihrer Hydrate vorliegen.

Die erfindungsgemäßen Verbindungen der allgemeinen Formel (I) können hergestellt werden, indem man reaktive Phenol-Derivate der allgemeinen Formel (II) mit reaktiven Phenylderivaten der allgemeinen Formel (III) 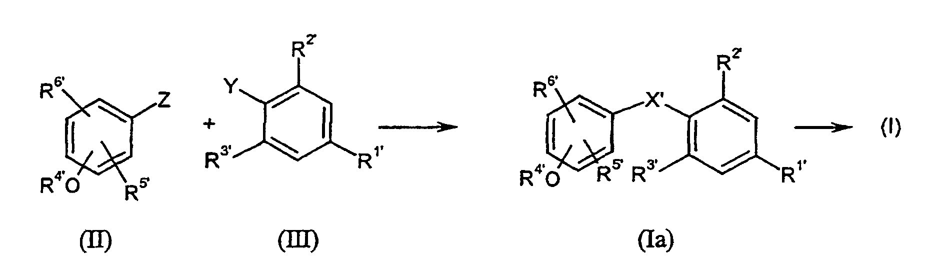 wobei die Substituenten R^{1'}, R^{2'}, R^{3'}, R^{4'}, R^{5'} und R^{6'} die oben für R¹ bis R⁶ angegebene Bedeutung haben und
Z und Y jeweils Gruppen entgegengesetzter Reaktivität darstellen, wobei z.B. Z ein elektrophiler Rest sein kann, der mit einem nucleophilen Y-Substituenten reagiert und vice versa,
X' die für X angegebene Bedeutung hat oder für 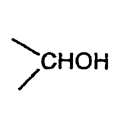 steht,
gegebenenfalls in Gegenwart von inerten Lösungsmitteln und Katalysatoren und gegebenenfalls unter Isolierung der Zwischenprodukte der allgemeinen Formel (Ia), in denen X' = -CH(OH)- bedeutet, oder direkt zu Verbindungen der Formel (I) umsetzt

Als Katalysatoren seien beispielhaft Kupplungskatalysatoren wie Pd- und/oder Cu-Verbindungen genannt.

Beispielhaft für die reaktiven Gruppen Z bzw. Y seien genannt: Formyl, CH₂Hal (Hal = Halogen, insbesondere Cl, Br oder I), CH₂OTosyl, Li, MgHal, Cu-,Pd-, Snoder Bor-Derivate.

Die erfindungsgemäß einsetzbaren Phenol-Derivate der allgemeinen Formel (II) sind bekannt oder können nach bekannten Methoden hergestellt werden (vergleiche z.B. Chemistry & Biology 1998, 5, 299-306, J. Org. Chem. 1956, 21, 1458)

Die Phenyl-Derivate der allgemeinen Formel (III) sind ebenfalls bekannt oder können nach bekannten Methoden hergestellt werden (vergleiche z.B. J. Med. Chem. 1997, **40,** 3542-3550 sowie die oben für die Phenol-Derivate der Formel (II) angegebenen Literatursiellen).

Die Umsetzung der Ausgangsverbindungen (II) mit (III) verläuft im allgemeinen bei Normaldruck. Sie kann aber auch unter erhöhtem oder reduziertem Druck durchgeführt werden.

Die Reaktion kann in einem Temperaturbereich von -100°C bis 200°C, vorzugsweise zwischen -78°C und 150°C in Gegenwart von inerten Lösunngsmitteln durchgeführt werden. Als inerte Lösungsmittel seien vorzugsweise genannt: Dimethylsulfoxid (DMSO), Dimethylformamid (DMF), Tetrahydofuran (THF), Diethylether etc.

Je nach spezifischem Substituentenmuster können bei der Umsetzung von (II) und (III) auch Zwischenprodukte der Formel (Ia) entstehen, in denen X' für eine CHOH-Gruppe steht, die dann mit oder ohne Isolierung dieser Zwischenstufen nach üblichen Methoden zu der entsprechenden Methylengruppe reduziert werden kann

Das erfindungsgemäße Verfahren kann durch folgende Formelschemata beispielhaft erläutert werden:

### Verfahrensvariante (A)

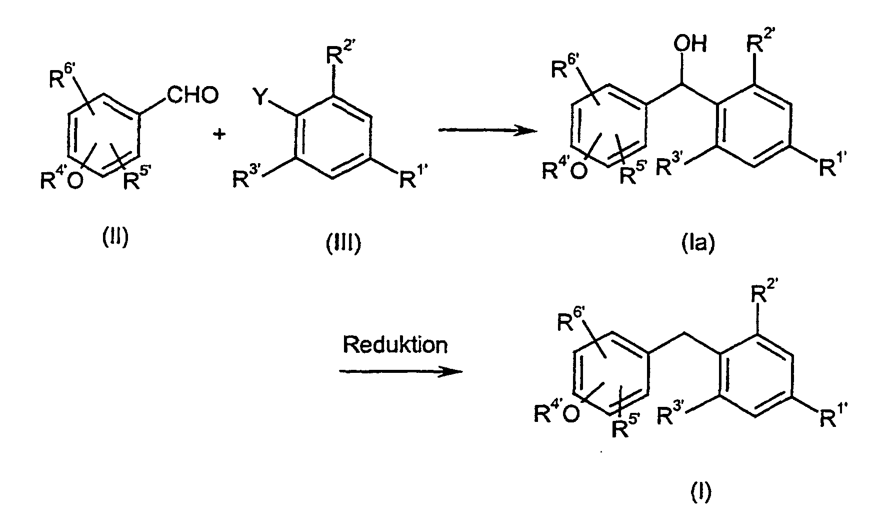

### Verfahrensvariante (B)

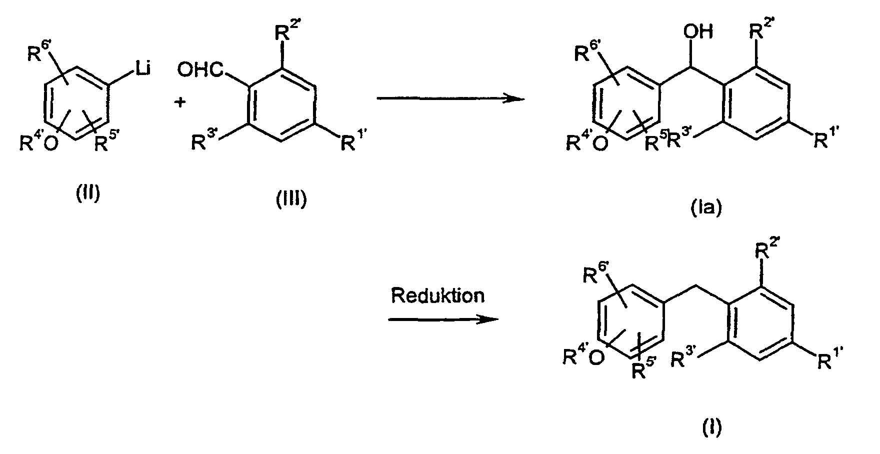

Je nach Bedeutung der Substituenten R^{1'}, R^{2'}, R^{4'}, R^{5'}, und R^{6'} kann es sinnvoll oder erforderlich sein, diese auf einzelnen Verfahrensstufen im angegebenen Bedeutungsumfang zu variieren. Auch kann es erforderlich sein, bestimmte Substituenten intermediär mit üblichen Schutzgruppen zu schützen und sie auf einer späteren Stufe durch Abspaltung der Schutzgruppe wieder freizusetzen.

Unter Schutzgruppen werden in der vorliegenden Anmeldung solche Gruppen in Ausgangs-, Zwischen- und/oder Endprodukten verstanden, die anwesende funktionelle Gruppen wie z.B. Carboxyl-, Amino- oder Hydroxygruppen schützen und die in der präparativen organischen Chemie üblich sind. Die so geschützten Gruppen können dann in einfacher Weise unter bekannten Bedingungen in freie funktionelle Gruppen umgewandelt werden.

Die bei der Umsetzung von (II) und (III) auftretenden Vorstufen bzw. Zwischenprodukte der Formel (Ia), in denen X' für -CH(OH)- steht, sind ebenfalls Gegenstand dieser Erfindung. Sie besitzen wesentliche Strukturmerkmale der erfindungsgemäßen Verbindungen der Formel (I) und ihrer Prodrugs und erfüllen somit als neue Zwischenprodukte alle Patentierungsvoraussetzungen.

Die erfindungsgemäßen Verbindungen der Formel (I) zeigen ein überraschendes und wertvolles pharmakologisches Wirkungsspektrum und lassen sich daher als vielseitige Medikamente einsetzen. Insbesondere lassen sie sich bei allen Indikationen einsetzen, die mit natürlichen Schilddrüsenhormonen behandelt werden können, wie beispielhaft und vorzugsweise Depression, Kropf oder Schilddrüsenkrebs. Bevorzugt lassen sich mit den erfindungsgemäßen Verbindungen der Formel (I) Arteriosklerose, Hypercholesterolämie und Dyslipidämie behandeln. Darüber hinaus lassen sich auch Fettsucht und Fettleibigkeit (Obesity) und Herzinsuffiziens behandeln und eine postprandiale Senkung der Triglyceride erreichen.

Die Verbindungen eignen sich auch zur Behandlung bestimmter Atemwegserkrankungen und zwar insbesondere von Lungenemphysem und zur medikamentösen Förderung der Lungenreifung.

Die Verbindungen eignen sich weiterhin zur Behandlung der Alzheimer'schen Krankheit.

Die Verbindungen eignen sich weiterhin zur Behandlung von Osteoporose, Herzrhythmusstörungen, Hypothyroidismen und Hauterkrankungen.

Außerdem lassen sich die Verbindungen auch zu Förderung und Regeneration des Haarwachstums einsetzen.

Die erfindungsgemäßen Wirkstoffe eröffnen eine weitere Behandlungsaltemative und stellen eine Bereicherung der Pharmazie dar. Im Vergleich zu den bekannten und bisher eingesetzten Schilddrüsenhormonpräparaten zeigen die erfindungsgemäßen Verbindungen ein verbessertes Wirkungsspektrum. Sie zeichnen sich vorzugsweise durch große Spezifität, gute Verträglichkeit und geringere Nebenwirkungen insbesondere im Herz-Kreislauf-Bereich aus.

Ihre Wirksamkeit lässt sich z.B. in-vitro durch den folgenden T 3 Promoter Assay-Zelltest prüfen:

Der Test wird mit einer stabil transfizierten, humanen HepG2 Hepatocarcinomzelle duchgeführt, die ein Luciferase-Gen unter der Kontrolle eines Thyroidhormon - regulierten Promoters exprimiert. Der zur Transfektion verwendete Vektor trägt vor dem Luciferase-Gen einen minimalen Thymidin-Kinase-Promoter mit einem Thyroidhormon - responsiven Element (TRE) das aus zwei invertierten Palindromen von je 12 Bp und einem 8Bp Spacer besteht.

Zum Test werden die Zellkulturen in 96 well-Platten ausgesät in Eagle's Minimal Essential Medium mit folgenden Zusätzen: Glutamin, Tricine, Natriumpyruvat, nicht-essentielle Aminosäuren, Insulin, Selen und Transferrin. Bei 37°C und 10 % CO₂-Atmosphär werden die Kulturen 48 Stunden angezüchtet. Dann werden serielle Verdünnungen von Testsubstanz oder Referenzverbindung (T3, T4) und Kostimulator Retinolsäure zu den Testkulturen gegeben und diese für weitere 48 oder 72 Stunden wie zuvor inkubiert. Jede Substanzkonzentration wird in vier Replikaten getestet. Zur Bestimmung der durch T3 oder andere Substanzen induzierten Luciferase werden die Zellen anschließend durch Zugabe eines Triton- und Luciferinhaltigen Puffers lysiert und sofort luminometrisch gemessen. Die EC₅₀-Werte jeder Verbindung werden berechnet (siehe Tabelle 1).

**Tabelle 1**

| **Beispiel** | **EC**_{**50**} **[nM]** |
|---|---|
| 2 | 0,14 |
| 4 | 0,23 |
| 5 | 4,3 |

Auch in dem in folgenden beschriebenen in-vivo Test zeigen die erfindungsgemäßen Verbindungen überraschend vorteilhafte Eigenschaften:

Testbeschreibung zur Auffindung von pharmakologisch wirksamen Substanzen, die das Serumcholesterin bei Mäusen senken.

Die Substanzen, die auf ihre serumcholesterinsenkende Wirkung in vivo untersucht werden sollen, werden männlichen Mäusen mit einem Körpergewicht zwischen 25 und 35 g oral verabreicht. Die Tiere werden einen Tag vor Versuchsbeginn in Gruppen mit gleicher Tierzahl, in der Regel n = 7-10, eingeteilt. Während des gesamten Versuches steht den Tieren Trinkwasser und Futter ad libitum zur Verfügung.
Die Substanzen werden einmal täglich 7 Tage lang oral verabreicht. Zu diesem Zwecke werden die Testsubstanzen in einer Lösung aus Solutol HS 15 + Ethanol + Kochsalzlösung (0,9 %) im Verhältnis 1 + 1 + 8 oder in einer Lösung aus Solutol HS 15 + Kochsalzlösung (0,9 %) im Verhältnis 2 + 8 gelöst. Die Applikation der gelösten Substanzen erfolgt in einem Volumen von 10 ml/kg Körpergewicht mit einer Schlundsonde. Als Kontrollgruppe dienen Tiere, die genauso behandelt werden, aber sie erhalten nur das Lösungsmittel (10 ml/kg Körpergewicht) ohne Testsubstanz.

Vor der ersten Substanzapplikation wird jeder Maus zur Bestimmung des Serumcholesterins Blut durch Punktion des retroorbitalen Venenplexus entnommen (Vorwert). Anschließend wird den Tieren mit einer Schlundsonde die Testsubstanz zum ersten Mal verabreicht. 24 Stunden nach der letzten Substanzapplikation, (am 8. Tag nach Behandlungsbeginn), wird jedem Tier zur Bestimmung des Serumcholesterins erneut Blut durch Punktion des retroorbitalen Venenplexus entnommen. Die Blutproben werden zentrifugiert und nach Gewinnung des Serums wird das Cholesterin photometrisch mit einem EPOS Analyzer 5050 (Eppendorf-Gerätebau, Netheler & Hinz GmbH, Hamburg) bestimmt. Die Bestimmung erfolgt mit einem handelsüblichen Enzymtest (Boehringer Mannheim, Mannheim).

Die Wirkung der Testsubstanzen auf die Serumcholesterin-Konzentration wird durch Subtraktion des Cholesterinwertes der 1. Blutentnahme (Vorwert) von dem Cholesterinwert der 2. Blutentnahme (nach Behandlung) bestimmt. Es werden die Differenzen aller Cholesterinwerte einer Gruppe gemittelt und mit dem Mittelwert der Differenzen der Kontrollgruppe verglichen.

Die statistische Auswertung erfolgt mit Student's t-Test nach vorheriger Überprüfung der Varianten auf Homogenität.

Substanzen, die das Serumcholesterin der behandelten Tiere, verglichen mit dem der Kontrollgruppe, statistisch signifikant (p < 0,05) um mindestens 10 % erniedrigen, werden als pharmakologisch wirksam angesehen.

Für die Applikation der Verbindungen der allgemeinen Formel (I) kommen alle üblichen Applikationsformen in Betracht, d.h. also oral, parenteral, inhalativ, nasal, sublingual, rektal oder äußerlich wie z.B. transdermal, insbesondere bevorzugt oral oder parenteral. Bei der parenteralen Applikation sind insbesondere intravenöse, intramuskuläre, subkutane Applikation zu nennen, z.B. als subkutanes Depot. Ganz besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird. Im allgemeinen kann der Wirkstoff in einer Konzentration von 0,1 bis 100 Gew.-%, insbesondere 0,5 bis 90 Gew.-%, vorzugsweise 5 bis 80 Gew.-%, vorliegen. Insbesondere sollte die Konzentration des Wirkstoffs 0,5 - 90 Gew.-% betragen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten selbstverständlich auch Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserern oder Farbstoffen versetzt werden.

Bei oraler Applikation werden vorzugsweise Dosierungen von 0,001 bis 5 mg/kg, vorzugsweise 0,005 bis 3 mg/kg Körpergewicht je 24 Stunden appliziert.

Die neuen Wirkstoffe können alleine und bei Bedarf auch in Kombination mit anderen Wirkstoffen vorzugsweise aus der Gruppe CETP-Inhibitoren, Antidiabetika, Antioxidantien, Cytostatika, Calciumantagonisten, Blutdrucksenkende Mittel, Thyroidhormone, Inhibitoren der HMG-CoA-Reduktase-Genexpression, Inhibitoren der HMG-CoA-Reduktase, Squalensynthese-Inhibitoren, ACAT-Inhibitoren, durchblutungsfördernde Mittel, Thrombozytenaggregationshemmer, Antikoagulantien, Angiotensin-II-Rezeptor-Antagonisten, Cholesterin-Absorptionshemmer, MTP-Inhibitoren, Fibrate, Niacin und PPAR-Agonisten verabreicht werden.

Die nachfolgenden Ausführungsbeispiele sollen die Erfindung exemplarisch erläutern ohne beschränkende Wirkung auf den Schutzbereich.

**Ausführungsbeispiele** (Ausgangsverbindungen)

### Beispiel I

### 2,6-Dimethyl-4-nitrophenyl trifluormethansulfonat

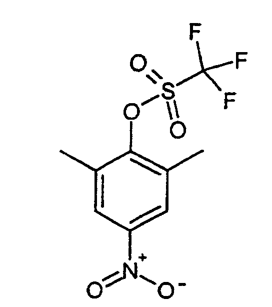

Eine Lösung von 150 g (0.9 mol) Dimethylnitrophenol in 700 ml Dichlormethan wird auf -15°C gekühlt. 181 ml Pyridin werden zugegeben. 308.8 g (1.09 mol) Trifluormethansulfonsäureanhydrid werden nun tropfenweise über einen Zeitraum von 2 h so zudosiert, dass die Reaktionstemperatur -5°C nicht übersteigt. Bei einer Temperatur von -5°C werden dann 150 ml Wasser zugesetzt. Die organische Phase wird nacheineander mit 3-5°C kalter HCl (1 molar ca. 300 ml ) und kaltem Wasser (2 mal) nacheinander ausgeschüttelt. Das Lösungsmittel wird im Vakuum entfernt und der Rückstand mit Petrolether verrührt. Der Niederschlag wird abgesaugt. Durch Kühlen der Mutterlauge erhält man eine weitere Fraktion. Ausbeute: 232 g (85 %)

¹H-NMR (200 MHz, CDCl₃): 2.50, s, 6H; 8.04, s, 2H.

### Beispiel II

### 2-Brom-1,3-dimethyl-5-nitrobenzol

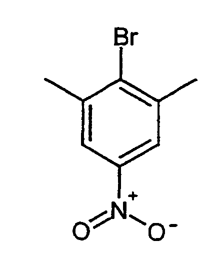

10.7 g (35.8 mmol) 2,6-Dimethyl-4-nitrophenyl-trifluormethansulfonat (Beispiel I) und 4.84 (55.8 mmol) LiBr werden bei 120°C in 120 ml N-Methylpyrrolidinoin 41 h gerührt. (Lösungsmittel: Ethylacetat/Cyclohexan 1:4). Nach dem Abkühlen wird langsam mit 80 ml Wasser versetzt und 1h unter Eisbadkühlung gerührt. Der Niederschlag wird abgesaugt, und mit 200 ml Petrolether verrührt. Das Lösungsmittel wird im Vakuum entfernt. Man erhält 7.23 g (85 %) Feststoff.

¹H-NMR (200 MHz, DMSO-D₆): 2.52, s, 6H; 8.02, s, 2H.

### Beispiel III

### 4-Brom-3,5-dimethylanilin

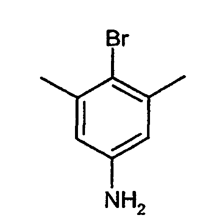

153 g Zinndichloriddihydrat (678 mmol) werden zu 1.71 konz. HCl gegeben und auf ca. 50°C erhitzt. Man gibt 26 g (113 mmol) 2-Brom-1,3-dimethyl-5-nitrobenzol (Beispiel II) zu. Die Suspension wird 20 min auf ca. 70°C erhitzt. Anschließend läßt man langsam auf Raumtemperatur abkühlen. Der ausgefallene weiße Feststoff wird abfiltriert und mit kaltem Aceton nachgewaschen. Das erhaltene Hydrochlorid wird in 1 N NaOH aufgenommen, kurz ausgerührt und mit Ethylacetat 5 mal extrahiert. Die organischen Phasen werden vereinigt, über Natriumsulfat getrocknet und das Lösungsmittel im Vakuum entfernt.

¹H-NMR (200 MHz, DMSO-D₆): 2.19, s, 6H; 5.21, s, breit, 2H; 6.39, s, 2H

### Beispiel IV

### tert-Butyl 4-brom-3,5-dimethylphenylcarbamat

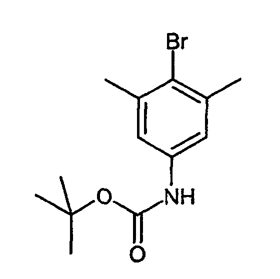

4 g (14.9 mmol) 4-Brom-3,5-dimethylanilin (Beispiel III) und 4.2 g (19.4 mmol) Pyrokohlensäure-di-tert.-butylester werden in 150 ml THF 5 Stunden am Rückfluß erhitzt. Das Lösungsmittel wird im Vakuum entfernt, der Rückstand in Ether aufgenommen und die Lösung nacheinander mit 10 %iger Zitronensäurelösung, Natriumhydrogencarbonatlösung und Wasser gewaschen. Die organische Phase wird über Natriumsulfat getrocknet und das Lösungsmittel im Vakuum entfernt. Man erhält 4.3 g (95 %) tert-Butyl 4-brom-3,5-dimethylphenylcarbamat.

¹H-NMR (200 MHz, DMSO-D₆): 1.47, s, 9H; 2.28, s, 6H; 7.29, s, 2H; 9.39, s, 1H.

### Beispiel V

### tert-Butyl 4-[hydroxy(4-methoxy-3-isopropylphenyl)methyl]-3,5-dimethylphenylcarbamat

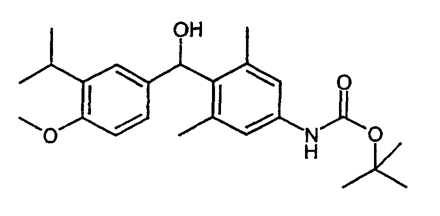

970 mg (3.23 mmol) tert-Butyl 4-brom-3,5-dimethylphenylcarbamat (Beispiel IV) werden in 3 ml Cyclohexan und 3 ml Ether gelöst, auf -78°C gekühlt und zu 2.53 ml 1.6 M Methyllithium in Ether bei -78°C getropft. Man rührt bei dieser Temperatur 10 Minuten und tropft dann 3.8 ml 1.7 M tert-Butyllithium in Pentan zu. Man rührt 1 Stunde bei -78°C, gibt 518 mg (2.9 mmol) 3-Isopropyl-4-methoxybenzaldehyd, gelöst in 1 ml Ether/Cyclohexan (1/1) zu, rührt 30min bei -78°C und eine Stunde bei Raumtemperatur. Man verdünnt mit Wasser und Ether, wäscht die organische Phase mit 10%iger Zitronensäurelösung, Natriumhydrogencarbonatlösung und Wasser, trocknet über Natriumsulfat und entfernt das Lösungsmittel im Vakuum. Chromatographische Reinigung (Cyclohexan/Ethylacetat = 9:1) ergibt 0.76 g (53 %) tert-Butyl 4-[hydroxy(4-methoxy-3-isopropylphenyl)methyl]-3,5-dimethylphenylcarbamat.

¹H-NMR (400 MHz, CDCl₃): 1.16, d, 3H; 1.20, d, 3H; 1.52, s, 9H; 2.05, d, 1H; 2.25, s, 6H; 3.28, hept, 1H; 3.81, s, 3H; 6.26, d, 1H; 6.40, s, 1H; 6.72, m, 1H; 6.90, dd, 1H; 7.06, s, 2H; 7.22,m, 1H.

### Beispiel VI

### tert-Butyl 4-(4-methoxy-3-isopropylbenzyl)-3,5-dimethylphenylcarbamat

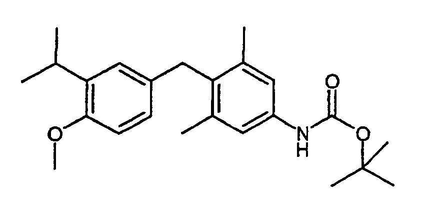

730 mg (1.66 mmol) tert-Butyl 4-[hydroxy(4-methoxy-3-isopropylphenyl)methyl]-3,5-dimethylphenyl-carbamat (Beispiel V) werden mit 700 mg Pd/C (10 %) 4 Stunden bei RT mit 1 bar Wasserstoff hydriert. Man filtriert über Kieselgur, entfernt das Lösungsmittel im Vakuum und erhält nach chromatographischer Reinigung 578 mg (91 %) tert-Butyl 4-(4-methoxy-3-isopropylbenzyl)-3,5-dimethylphenylcarbamat.

¹H-NMR (400 MHz, CDCl₃): 1.18, d, 6H; 1.52, s, 9H; 2.21, s, 6H; 3.26, hept, 1H; 3.78, s, 3H; 3.92, s, 2H; 6.38, s, 1H; 6.63, m, 2H; 6.95, m, 1H; 7.08, s, 2H.

### Beispiel VII

### 4-(3-Isopropyl-4-methoxybenzyl)-3,5-dimethylanilin

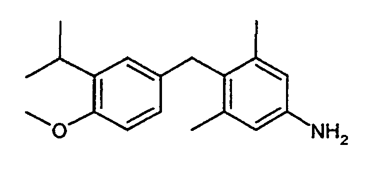

578 mg (1.36 mmol) tert-Butyl 4-(4-methoxy-3-isopropylbenzyl)-3,5-dimethylphenylcarbamat (Beispiel VI) werden in 10 ml 5 % Trifluoressigsäure in Dichlormethan bei 0°C gelöst und 4 Stunden gerührt. Man neutralisiert mit Natriumhydrogencarbonatlösung und trocknet über Natriumsulfat. Nach dem Entfernen des Lösungsmittels wird ohne weitere Reinigung weiter umgesetzt.

¹H-NMR (400 MHz, CDCl₃): 1.16, d, 6H; 2.16, s, 6H; 3.26, hept, 1H; 3.78, s, 3H; 3.89, s, 2H; 6.45, m, 1H; 6.68, m, 1H; 6.95, s, 1H.

### Beispiel VIII

### tert-Butyl 4-[[3-(4-fluorbenzyl)-4-methoxyphenyl](hydroxy)methyl]-3,5-dimethylphenylcarbamat

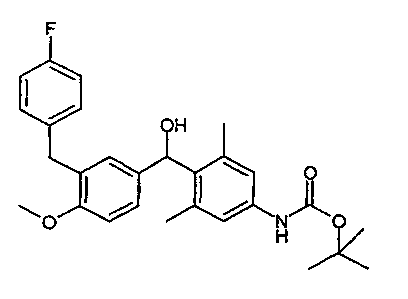

1.3 ml Methyllithium (2.1 mmol, 1.6 M in Diethylether) werden in 1 ml Diethylether vorgelegt und mit 600 mg (1.99 mmol) tert-Butyl 4-brom-3,5-dimethylphenylcarbamat (Beispiel IV) in 2 ml Diethylether/1 ml THF bei -78°C versetzt. Nach 20 min wurde 1.53 ml t-BuLi (2.6 mmol, 1.7 M in Pentan) zugetropft und 30 min bei -78° gerührt. 3-(4-Fluorbenzyl)-4-methoxybenzaldehyd wird, gelöst in 3 ml THF, zugetropft. Man rührt 1 h bei -78°C und gibt dann wäßrige NH₄Cl-Lösung zu. Es wird mit Diethylether verdünnt, mit Wasser extrahiert, die organische Phase getrocknet und einrotiert. Chromatographie (Cyclohexan/Ethylacetat=6:1) ergibt 493 mg (46 %) *tert*-Butyl 4-[[3-(4-fluorbenzyl)-4-methoxyphenyl](hydroxy)methyl]-3,5-dimethylphenylcarbamat.

¹H-NMR (300 MHz, CDCl₃): 1.52, s, 9H; 2.21, s, 6H; 3.78, s, 3H; 3.90, d, 2H; 6.20, d, breit, 1H; 6.38, s, breit, 1H; 6.77, d, 1H; 6.90, m, 4H; 7.05, s, 2H; 7.10, m, 3H.

### Beispiel IX

### tert-Butyl 4-[3-(4-fluorbenzyl)-4-methoxybenzyl]-3,5-dimethylphenylcarbamat

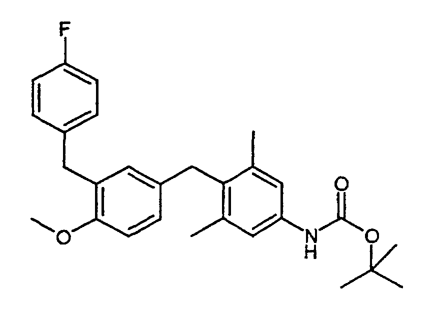

590 mg *tert-*Butyl 4-[[3-(4-fluorbenzyl)-4-methoxyphenyl](hydroxy)methyl]-3,5-dimethylphenyl-carbamat (Beispiel VIII), 24 mg Pd/Kohle (10 %) und ein Tropfen Essigsäure werden in 10 ml Methanol mit Wasserstoff bei Normaldruck 4 h hydriert. Die Suspension wird über Kieselgur filtriert Das Filtrat wird am Rotationsverdampfer eingeengt. Chromatographie (Cyclohexan/Ethylacetat = 6:1.) ergibt 449 mg (77 %) *tert-*Butyl 4-[3-(4-fluorbenzyl)-4-medioxybenzyl]-3,5-dimethylphenylcarbamat.

¹H-NMR (300 MHz, CDCl₃): 1.52, s, 9H; 2.19, s, 6H; 3.72, s, 3H; 3.85, d, 4H; 6.72, m, 3H; 6.92, m, 2H; 7.05, m, 4H.

### Beispiel X

### 4-[3-(4-Fluorbenzyl)-4-methoxybenzyl)-3,5-dimethylanilin

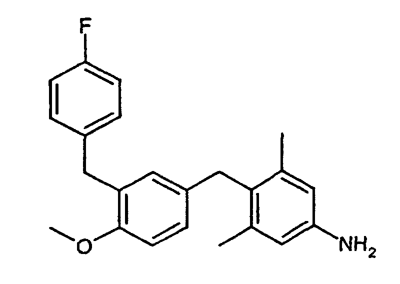

460 mg (1.02 mmol) *tert*-Butyl 4-[3-(4-fluorbenzyl)-4-methoxybenzyl]-3,5-dimethylphenylcarbamat (Beispiel IX) werden über Nacht in 10 ml Trifluoressigsäure/Dichlormethan (10 %ige Lösung) bei Raumtemperatur gerührt. Es wird mit NaHCO₃Lösung neutralisiert, die organische Phase getrennt, über Natriumsulfat getrocknet und einrotiert. Chromatographie (Cyclohexan/Ethylacetat = 6:1) ergibt 218 mg (55 %) 4-[3-(4-Fluorbenzyl)-4-methoxybenzyl]-3,5-dimethylanilin.

¹H-NMR (300MHz, CDCl₃): 2.22, s, 6H; 3.56, s, breit 2H; 3.74, s, 3H; 3.82, s, 2H; 3.85, s, 2H;

### Ausführungsbeispiele

### Beispiel 1

### Ethyl 2-[4-(3-isopropyl-4-methoxybenzyl)-3,5-dimethylanilin]-2-oxoacetat

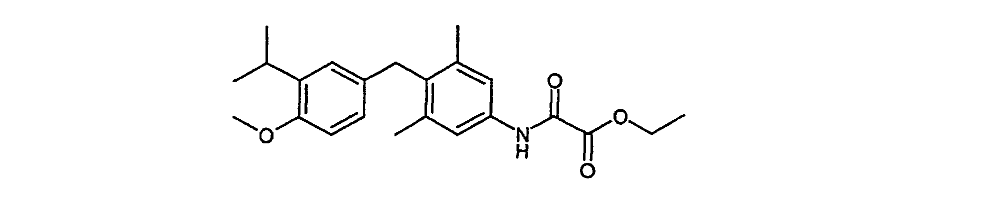

246 mg (0.86 mmol) 4-(3-Isopropyl-4-methoxybenzyl)-3,5-dimethylanilin (Beispiel VII) werden mit 97 mg (0.96 mmol) Triethylamin in 10 ml Dichlormethan gelöst und tropfenweise mit 160 mg (1.17 mmol) Oxalsäureesterchlorid versetzt. Man rührt 4 Stunden bei Raumtemperatur, wäscht mit Natriumhydrogencarbonatlösung und Wasser, trocknet über Natriumsulfat und entfernt das Lösungsmittel im Vakuum. Chromatographische Reinigung ergibt 128 mg (38 %) Ethyl 2-[4-(3-isopropyl-4-methoxybenzyl)-3,5-dimethylanilin]-2-oxoacetat.
R_{f}= 0.63 (Toluol/Acetonitril = 9:1).

### Beispiel 2

### 2-[4-(4-Hydroxy-3-isopropylbenzyl)-3,5-dimethylanilin]-2-oxoessigsäure

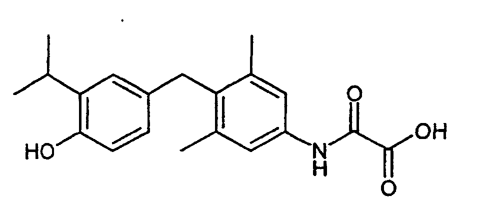

128 mg (0.33 mmol) Ethyl 2-[4-(3-isopropyl-4-methoxybenzyl)-3,5-dimethylanilin]-2-oxoacetat (Beispiel 1) werden in 5 ml Dichlormethan unter Argon bei -78°C tropfenweise mit 167 mg (0.66 mmol) Bortribromid versetzt. Die Reaktionsmischung wird 2.5 Stunden bei Raumtemperatur gerührt. Man gießt auf Puffer pH 7, trennt die Phasen, stellt die wäßrige Phase mit Puffer pH 4 sauer, extrahiert mit Dichlormethan und vereinigt die organischen Phasen. Es wird über Natriumsulfat getrocknet und das Lösungsmittel im Vakuum entfernt. Man erhält 112 mg (98 %) 2-[4-(4-Hydroxy-3-isopropylbenzyl)-3,5-dimethylanilin]-2-oxoessigsäure.

¹H-NMR (400 MHz, CDCl₃): 1.21, d, 6H; 2.26, s, 6H; 3.15, hept, 1H; 3.95, s, 2H; 4.52, s, breit, 1H; 6.57, m, 2H; 6.91, m, 1H; 7.32, s, 2H; 8.83, s, breit, 1H.

### Beispiel 3

### Ethyl-({4-[3-(4-fluorbenzyl)-4-methoxybenzyl]-3,5-dimethylphenyl}amino)(oxo)-acetat

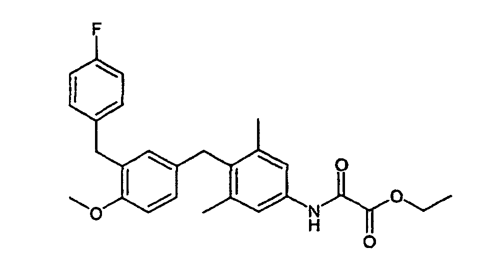

335 mg (0.96 mmol) 4-[3-(4-Fluorbenzyl)-4-methoxybenzyl]-3,5-dimethylanilin (Beispiel X) und 107 mg (1.06 mmol) Triethylamin werden in ml Dichlormethan vorgelegt und bei 0°C tropfenweise mit 177 mg (1.29 mmol) Oxalsäureethylesterchlorid versetzt. Man läßt 4 h rühren. Man schüttelt mit NaHCO₃-Lösung und NaCl-Lösung, trocknet die organische Phase und rotiert ein. Chromatographische Reinigung (Cyclohexan/Ethylacetat = 7:1) ergibt 231 mg Ethyl-({4-[3-(4-fluorbenzyl)-4-methoxybenzyl]-3,5-dimethylphenyl}amino)(oxo)acetat.

¹H-NMR (400 MHz, DMSO-D₆): 1.21, t, 3H; 2.21, s, 6H; 3.42, quart, 2H; 3.74, s, 4H; 6.71, m, 2H; 6.92, m, 3H; 7.10, m 2H; 7.31,s, 2H; 8.73, s, 1H.

### Beispiel 4

### ({4-[3-(4-Fluorbenzyl)-4-bydroxybenzyl]-3,5-dimethylphenyl}amino)(oxo)essigsäure

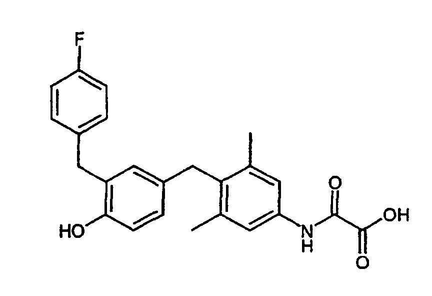

102 mg (0.22 mmol) Ethyl-({4-[3-(4-fluorbenzyl)-4-methoxybenzyl]-3,5-dimethylphenyl-}amino)(oxo)-acetat (Beispiel 3) werden in 5 ml Dichlormethan unter Argon bei -78°C langsam mit 65 mg (0.25 mmol) BBr₃ versetzt. Es wird über Nacht gerührt, während die Reaktionsmischung Raumtemperatur erreicht. Man kühlt wieder auf -78°C und gibt weitere mit 65 mg (0.25 mmol) BBr₃ zu. Es wird 2 Stunden bei Raumtemperatur gerührt. Man gießt auf Eiswasser, rührt 2 Stunden, extrahiert mit Dichlormethan und Ethylacetat, torcknet über Natriumsulfat und rotiert ein. Der Rückstand wird mit Ether verrührt und der Feststoff abgesaugt. Man erhält 51 mg (48%) ({4-[3-(4-Fluorbenzyl)-4-hydroxybenzyl]-3,5-dimethylphenyl}amino)(oxo)-essigsäure.

¹H-NMR (400 MHz, DMSO-D₆): 2.12, s, 6H; 3.78,s, 4H; 6.61, m, 4H; 7.05, m, 2H; 7.18, m, 2H; 7.49, s, 2H; 9.20, s, 1H; 9.91, s, 1H.

### Beispiel 5

### ({4-[3-(4-Fluorbenzyl)-4-methoxybenzyl]-3,5-dimethylphenyl} amino)(oxo)essigsäure

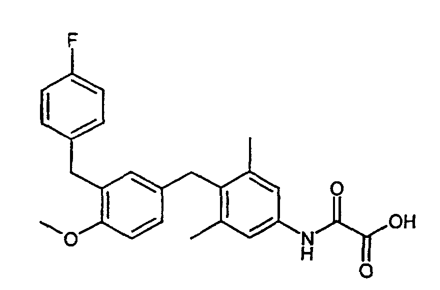

290 mg (0.64 mmol) Ethyl-({4-[3-(4-fluorbenzyl)-4-methoxybenzyl)-3,5-dimethylphenyl-}amino)(oxo)-acetat (Beispiel 3) werden in 8 ml Dichlormethan unter Argon bei -78°C langsam mit 356 mg (1.41 mmol) BBr₃ versetzt. Man läßt die Mischung auf Raumtemperatur auftauen und rührt 2,5 h. Man gießt die Reaktionsmischung auf Puffer pH 7, trennt die organische Phase ab und extrahiert die wässrige Phase nach Einstellen des pH-Wertes auf pH 7. Die vereinigten organischen Phasen werden mit gesättigter NaCl-Lösung gewaschen getrocknet und einrotiert. Der Rückstand wird mit Diethylether verrührt, abgesaugt und getrocknet.

¹H-NMR (300 MHz, CDCl₃): 2.22, s, 6H; 3.75, s, 3H; 3.85, s, 2H; 3.92, s, 2H; 6.61, m, 3H; 6.91, m, 2H; 7.09, m, 2H; 7.30, s, 2H; 8.82, s, 1H.

## Patentansprüche

1. Verbindungen der allgemeinen Formel (I) 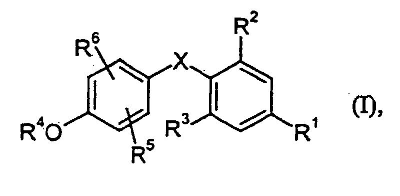 worin
X CH₂, CHF oder CF₂ bedeutet,
R¹ für eine Gruppe der Formet -NH-C(O)-C(O)-OR¹¹ steht, worin R¹¹ Wasserstoff oder (C₁-C₄)-Alkyl bedeutet,
R² und R³ gleich oder verschieden sind und für Halogen, (C₁-C₄)-Alkyl, Cyclopropyl, CF₃, CHF₂ oder CH₂F stehen,
R⁴ Wasserstoff oder (C₁-C₃)-Alkyl bedeutet,
R⁵ Wasserstoff bedeutet,
und
R⁶ für (C₁-C₆)-Alkyl oder (C₃-C₆)-Cycloalkyl steht oder für einen Rest der Formel 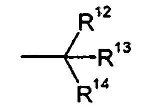 steht, worin
R¹² für Phenyl, Pyrimidinyl, Pyridyl oder 3(2*H*)-Pyridazinonyl, die jeweils bis zu dreifach gleich oder verschieden durch Halogen, Hydroxy, Trifluormethyl, (C₁-C₃)-Alkyl oder (C₁-C₃)-Alkoxy substituiert sein können, oder für einen Rest der Formel -NR¹⁸R¹⁹ steht,
worin
R¹⁸ und R¹⁹ gleich oder verschieden sind und unabhängig voneinander für (C₁-C₄)-Alkyl stehen, das durch Hydroxy, Amino, Mono- oder Di-(C₁-C₄)-alkylamino, oder durch Phenyl, Pyridyl oder Pyrimidinyl, die jeweils gegebenenfalls bis zu dreifach durch Hydroxy, Halogen, Trifluor-methyl, Methoxy oder (C₁-C₃)-Alkyl substituiert sind, substituiert sein kann,
R¹³ und R¹⁴ gemeinsam für eine Oxo-Gruppe stehen oder gleich oder verschieden sind und Wasserstoff, Hydroxy, (C₁-C₆)-Alkyl, bedeuten,
und deren Salze.

2. Verfahren zur Herstellung von Verbindungen der allgemeinen Formel (I) wie es Anspruch 1 definiert, **dadurch gekennzeichnet, dass** man reaktive Phenol-Derivate der allgemeinen Formel (II) mit reaktiven Phenylderivaten der allgemeinen Formel (III) 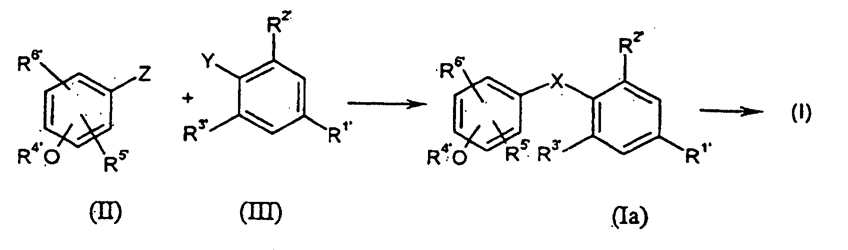 wobei die Substituenten R^{1'}, R^{2'}, R^{3'}, R^{4'}, R^{5'} und R^{6'} die in Anspruch 1 für R¹ bis R⁶ angegebenen Bedeutungen haben und
Z und Y jeweils Gruppen entgegengesetzter Reaktivität darstellen,
X' die für X in Anspruch 1 angegebene Bedeutung hat oder für 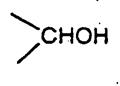 steht,
gegebenenfalls in Gegenwart von inerten Lösungsmitteln und Katalysatoren und gegebenenfalls unter Isolierung der Zwischenprodukte der allgemeinen Formel (Ia), in denen X' = -CH(OH)- bedeutet, oder direkt zu Verbindungen der Formel (I) umsetzt.

3. Verbindungen der allgemeinen Formel (Ia) 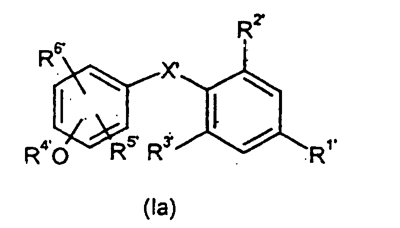 worin
R^{1'}, R^{2'}, R^{3'}, R^{4'}, R^{5'} und R^{6'} die in Anpruch 1 für R¹ bis R⁶ angegebenen Bedeutungen haben,
X' für -CH(OH)- steht
und deren Salze.

4. Arzneimittel enthaltend mindestens eine Verbindung der allgemeinen Formel (I) gemäß Anspruch 1.

5. Verfahren zur Herstellung von Arzheimitteln, **dadurch gekennzeichnet, dass** man mindestens eine Verbindung der allgemeinen Formel (I) gemäß Anspruch 1 mit Hilfs- und Trägerstoffen in eine geeignete Applikationsform überführt.

6. Verwendung der Verbindungen der allgemeinen Formel (I) gemäß Anspruch 1 zur Herstellung von Arzneimitteln.

7. Verwendung von Verbindungen der allgemeinen Formel (I) gemäß Anspruch 6 Herstellung von Arzneimitteln für die Behandlung und/oder Prophylaxe von Arteriosklerose und Hypercholesterolämie.

8. Verwendung von Verbindungen der allgemeinen Formel (I) gemäß Anspruch 6 zur Herstellung von Arzneimitteln für die Prophylaxe und/oder Behandlung von Krankheitsformen, die mit natürlichem Schilddrüsenhormon behandelt werden können.

9. Verwendung von Verbindungen der allgemeinen Formel (I) gemäß mindestens einem der Ansprüche 7 und 8 in Kombination mit anderen Arzneimitteln.

## Claims

1. Compounds of the general formula (I) 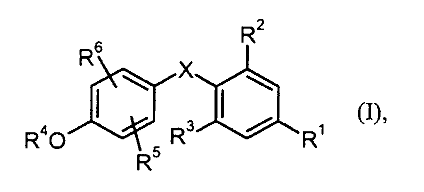 in which
X is CH₂, CHF or CF₂,
R¹ is a group of the formula -NH-C(O)-C(O)-OR¹¹, in which
R¹¹ is hydrogen or (C₁-C₄)-alkyl,
R² and R³ are identical or different and are halogen, (C₁-C₄)-alkyl, cyclo-propyl, CF₃, CHF₂ or CH₂F,
R⁴ is hydrogen or (C₁-C₃)-alkyl,
R⁵ is hydrogen,
and
R⁶ is (C₁-C₆)-alkyl or (C₃-C₆)-cycloalkyl or is a radical of the formula 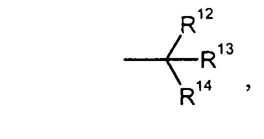 in which
R¹² is phenyl, pyrimidinyl, pyridyl or 3(2*H*)-pyridazinonyl, each of which may be substituted up to three times, identically or differently, by halogen, hydroxyl, trifluoromethyl, (C₁-C₃)- alkyl or (C₁-C₃)-alkoxy, or is a radical of the formula -NR¹⁸R¹⁹, in which
R¹⁸ and R¹⁹ are identical or different and are, independently of one another, (C₁-C₄)-alkyl which may be substituted by hydroxyl, amino, mono- or di-(C₁-C₄)- alkylamino, or by phenyl, pyridyl or pyrimidinyl, each of which is optionally substituted up to three times by hydroxyl, halogen, trifluoromethyl, methoxy or (C₁-C₃)-alkyl,
R¹³ and R¹⁴ together are an oxo group or are identical or different and are hydrogen, hydroxyl, (C₁-C₆)-alkyl,
and the salts thereof.

2. Process for preparing compounds of the general formula (I) as defined in Claim 1, **characterized in that** reactive phenol derivatives of the general formula (II) are reacted with reactive phenyl derivatives of the general formula (III) 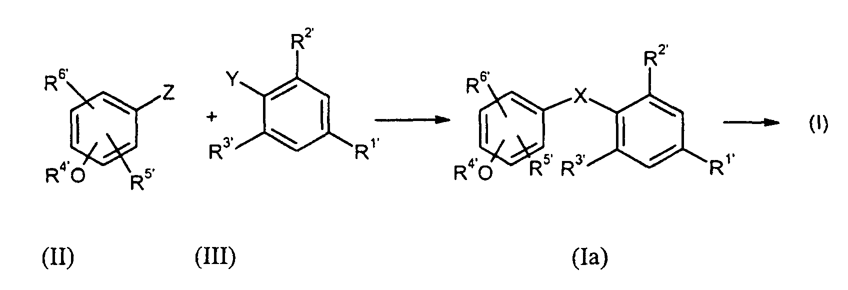 where the substituents R^{1'}, R^{2'}, R^{3'}, R^{4'}, R^{5'} and R^{6'} have the meanings stated for R¹ to R⁶ in Claim 1, and
Z and Y are each groups of opposite reactivity,
X' has the meaning stated for X in Claim 1 or is 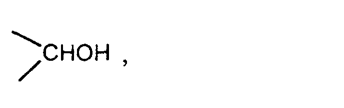 where appropriate in the presence of inert solvents and catalysts and, where appropriate, with isolation of the intermediates of the general formula (Ia)
in which X' is -CH(OH)-, or directly to compounds of the formula (I).

3. Compounds of the general formula (Ia) 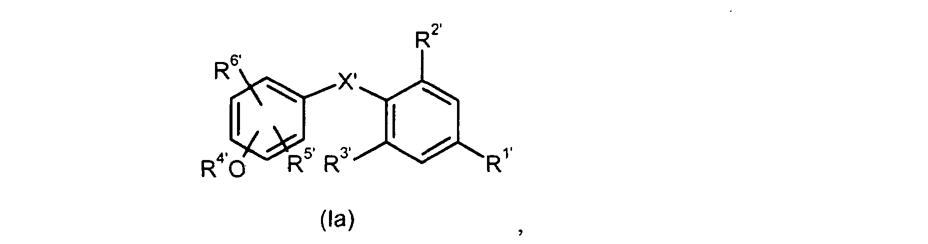 in which
R^{1'}, R^{2'}, R^{3'}, R^{4'}, R^{5'} and R^{6'} have the meanings stated for R¹ to R⁶ in Claim 1,
X' is -CH(OH)-,
and the salts thereof.

4. Medicament containing at least one compound of the general formula (I) according to Claim 1.

5. Process for producing medicaments, **characterized in that** at least one compound of the general formula (I) according to Claim I is converted with excipients and carriers into a suitable administration form.

6. Use of compounds of the general formula (I) according to Claim 1 for producing medicaments.

7. Use of compounds of the general formula (I) according to Claim 6 for producing medicaments for the treatment and/or prophylaxis of arteriosclerosis and hypercholesterolaemia.

8. Use of compounds of the general formula (I) according to Claim 6 for producing medicaments for the prophylaxis and/or treatment of types of disorders which can be treated with natural thyroid hormone.

9. Use of compounds of the general formula (I) according to at least one of Claims 7 and 8 in combination with other medicaments.

## Revendications

1. Composés de formule générale (I) 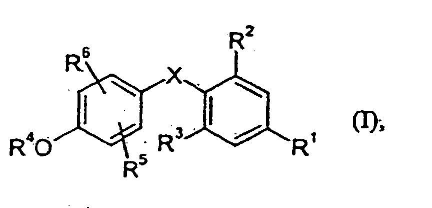 dans laquelle
X est un groupe CH₂, CHF ou CF₂,
R¹ est un groupe de formule -NH-C(O)-C(O)-OR¹¹ dans laquelle
R¹¹ représente l'hydrogène ou un reste alkyle en C₁ à C₄,
R² et R³ sont identiques ou différents et représentent un halogène, un reste alkyle en C₁ à C₄, cyclopropyle, CF₃, CHF₂ ou CH₂F,
R⁴ représente l'hydrogène ou un reste alkyle en C₁ à C₃,
R⁵ représente l'hydrogène,
et
R⁶ est un reste alkyle en C₁ à C₆ ou un reste cycloalkyle en C₃ à C₆ ou représente un reste de formule 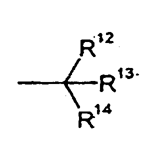 dans laquelle
R¹² est un reste phényle, pyrimidinyle, pyridyle ou 3(2*H*)-pyridazinonyle, chacun d'eux pouvant être substitués jusqu'à trois fois identiques ou différentes par un radical halogéno, hydroxy, trifluorométhyle, alkyle en C₁ à C₃ ou alkoxy en C₁ à C₃, ou représente un reste de formule -NR¹⁸R¹⁹, dans laquelle
R¹⁸ et R¹⁹ sont identiques ou différents et représentent, indépendamment l'un de l'autre un reste alkyle en C₁ à C₄ qui peut être substitué par un radical hydroxy, amino, mono- ou di (alkyle en C₁ à C₄) amino ou par un radical phényle, pyridyle ou pyrimidinyle dont chacun est éventuellement substitué jusqu'à trois fois par un radical hydroxy, halogéno, trifluorométhyle, méthoxy ou alkyle en C₁ à C₃,
R¹³ et R¹⁴ forment ensemble un groupe oxo ou sont identiques ou différents et représentent l'hydrogène, un groupe hydroxy, alkyle en C₁ à C₆,
et leurs sels.

2. Procédé de production de composés de formule générale (I) tel que défini dans la revendication 1, **caractérisé en ce qu'**on fait réagir des dérivés phénoliques réactifs de formule générale (II) avec des dérivés phényliques réactifs de formule générale (III) 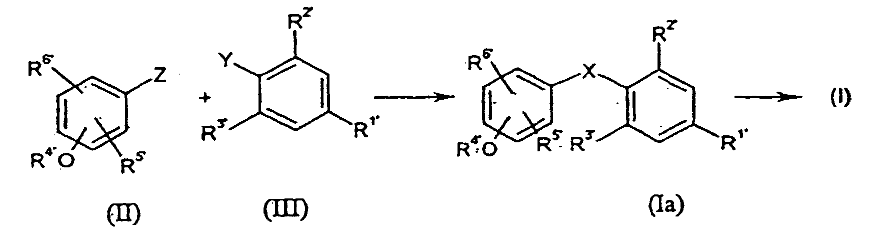 formules dans lesquelles
les substituants R^{1'}, R^{2'}, R^{3'}, R^{4'}, R^{5'} et R^{6'} ont les définitions indiquées pour R¹ à R⁶ dans la revendication 1 et
Z et Y représentent chacun des groupes de réactivité opposées,
X' a la définition indiquée pour X dans la revendication 1 ou représente un groupe 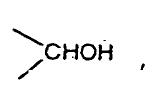 éventuellement en présence de solvants inertes et de catalyseurs et, le cas échéant, avec isolement des produits intermédiaires de formule générale (Ia), dans lesquels X' représente un groupe -CH(OH)-, ou bien directement pour obtenir des composés de formule (I).

3. Composés de formule générale (Ia) 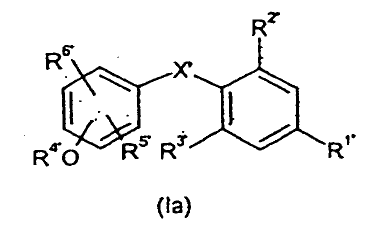 dans laquelle
R^{1'}, R^{2'}, R^{3'}, R^{4'}, R^{5'} et R^{6'} ont les définitions indiquées pour R¹ à R⁶ dans la revendication 1,
X' est un groupe -CH(OH)-
et leurs sels.

4. Médicament contenant au moins un composé de formule générale (I) suivant la revendication 1.

5. Procédé de préparation de médicaments, **caractérisé en ce qu'**on fait prendre à au moins un composé de formule générale (I) suivant la revendication 1, à l'aide de substances auxiliaires et de supports, une forme convenant pour l'administration.

6. Utilisation des composés de formule générale (I) suivant la revendication 1 pour la préparation de médicaments.

7. Utilisation de composés de formule générale (I) suivant la revendication 6 pour la préparation de médicaments destinés au traitement et/ou à la prophylaxie de l'artériosclérose et de l'hypercholestérolémie.

8. Utilisation de composés de formule générale (I) suivant la revendication 6 pour la préparation de médicaments destinés à la prophylaxie et/ou au traitement de pathologies qui peuvent être traitées avec l'hormone naturelle de la glande thyroïde.

9. Utilisation de composés de formule générale (I) suivant au moins l'une des revendications 7 et 8 en combinaison avec d'autres médicaments.
